# EUROPÄISCHE PATENTSCHRIFT

(11) **EP 3 558 443 B1**
(45) Veröffentlichungstag und Bekanntmachung des Hinweises auf die Patenterteilung: **22.11.2023**
(21) Anmeldenummer: 17826468.5
(22) Anmeldetag: 17.12.2017
(51) Int. Cl.: A61M 39/10, A61M 39/26, A61M 39/24, A61M 39/00, F16L 37/00, F16L 1/00, A61M 39/06, F16K 1/00

(54) **KUPPLUNGSKOMPONENTE UND FLUIDKUPPLUNG**
COUPLING COMPONENT AND FLUID COUPLING
ÉLÉMENT D'ACCOUPLEMENT ET RACCORD POUR FLUIDE

(30) Priorität: 21.12.2016 DE 102016015205
(43) Veröffentlichungstag der Anmeldung: 30.10.2019
(73) Patentinhaber: Fresenius Medical Care Deutschland GmbH, 61352 Bad Homburg v.d.H (DE)
(72) Erfinder: ANDERLE, Jens, 73326 Deggingen (DE); WIRTL, Hannes, 86956 Schongau (DE)
(74) Vertreter: Oppermann, Frank
(86) Internationale Anmeldenummer: PCT/EP2017/083183
(87) Internationale Veröffentlichungsnummer: WO 2018/114726

(56) Entgegenhaltungen:
- US-A- 5 064 416
- US-A1- 2005 256 460
- US-A1- 2013 165 868
- US-A1- 2014 276 462

## Beschreibung

Die Erfindung betrifft eine Fluidkupplung zur lösbaren Verbindung von fluidführenden Komponenten, insbesondere fluidführenden Komponenten in medizinischen Geräten, mit einer Kupplungskomponente sowie mit einer Kupplungseinrichtung .

Aus dem Stand der Technik ist eine Vielzahl von unterschiedlichen Fluidkupplungen bekannt, die eine lösbare Verbindung von fluidführenden Komponenten ermöglichen. Ein Beispiel für eine derartige Fluidkupplung zur lösbaren Verbindung von fluidführenden Komponenten im Bereich der Medizintechnik ist das Luer-System, das im medizinischen Bereich breite Anwendung findet.

Die US 2014/0276462 A1 beschreibt ein Kupplungssystem für einen intravenösen Katheter, das einen ersten Kupplungsteil und einen zweiten Kupplungsteil umfasst. In dem ersten Kupplungsteil ist ein Fluidkanal ausgebildet, in dem ein rohrförmiger Körper angeordnet ist, auf dem eine elastische Membran sitzt, die von einem Fluidspalt durchsetzt ist. Die Membran dichtet in einer Ruhestellung die Kanalöffnung des rohrförmigen Körpers ab und gibt in einer Funktionsstellung die Kanalöffnung frei. Das zweite Kupplungsteil ist als Luer-Konnektor ausgebildet. Wenn der Luer-Konnektor in den Fluidkanal des ersten Kupplungsteil vorgeschoben wird, schiebt dieses die Membran aus der Ruhestellung über den rohrförmigen Körper in die Funktionsstellung, in der sich die Membran derart verformt, dass der ringförmige Spalt zwischen der Innenwandung des Fluidkanals und der Außenwandung des rohrförmigen Körpers von der Membran vollständig ausgefüllt ist. Beim Einschieben des Luer-Konnektors stützt sich dieser allein an der elastischen Membran ab, so dass ein genau definierter Abstand nicht gegen ist. Die Anpresskraft auf die Membran hängt allein von der Kraft ab, mit der der Luer-Konnektor in das erste Kupplungsteil vorgeschoben wird.

Die US 5 064 416 A offenbart ein Kupplungssystem, bei dem eine elastische geschlitzte Membran mit einem Spike über einen rohrförmigen Körper in einem Fluidkanal eines Kupplungsteils geschoben wird, wodurch die elastische Membran gespreizt wird. In der Funktionsstellung sitzt die Membran nicht auf einer in dem Fluidkanal ausgebildeten Anlagefläche auf, die einen "zweiten Ventilsitz" schafft.

Aus der US 2005/256460 A1 ist ein Kupplungssystem bekannt, bei dem die elastische Membran in einem Kompartiment sitzt, in dem einen Kupplungsteil ausgebildet ist. In dem einzigen Kompartiment wird die *Membran* von allen Seiten umschlossen. Eine zusätzliche Ausnehmung zur Aufnahme eines Bereichs der Membran beim Übergang in die Funktionsstellung ist nicht vorgesehen.

Die Aufgabe der Erfindung besteht darin, eine Kupplungskomponente und eine Fluidkupplung bereitzustellen, die in vorteilhafter Weise gereinigt werden können und bei denen ein unerwünschtes Eindringen von Verunreinigungen in den Fluidkanal der Kupplungskomponente verhindert wird.

Diese Aufgabe wird für eine Fluidkupplung der eingangs genannten Art mit den Merkmalen des Patentanspruchs 1 gelöst. Die abhängigen Ansprüche betreffen vorteilhafte Ausführungsformen der Erfindung.

Bei der erfindungsgemäßen Fluidkupplung ist vorgesehen, dass die Kanalöffnung von einer Membrandichtung aus einem elastischen Material überdeckt ist, die von einem Fluidspalt durchsetzt ist, der derart ausgebildet ist, dass die Membrandichtung in einer Ruhestellung die Kanalöffnung abdichtet und einer Funktionsstellung die Kanalöffnung freigibt, wobei in der Kupplungsausnehmung benachbart zur Kanalöffnung wenigstens eine Ausweichvertiefung ausgebildet ist, die für eine Ausweichbewegung eines Bereichs der Membrandichtung bei einem Übergang der Membrandichtung aus der Ruhestellung in die Funktionsstellung ausgebildet ist.

Dabei kommt der Membrandichtung eine Doppelfunktion zu, da diese in der Ruhestellung die Kanalöffnung abdichtet und zudem ebenfalls in der Ruhestellung eine glatte und damit gut zu reinigende Oberfläche innerhalb der Kupplungsausnehmung bereitstellt. Ferner sind die Membrandichtung und die Kupplungsausnehmung derart aufeinander abgestimmt, dass diejenigen Bereiche der Membrandichtung, die bei einem Übergang aus der Ruhestellung in die Funktionsstellung aufgrund der dabei auftretenden elastischen Deformation der Membrandichtung eine Ausweichbewegung durchführen, möglichst ohne einen wesentlichen mechanischen Widerstand in die Funktionsstellung gebracht werden können.

Die Kupplungseinrichtung ist für eine aktive Bewegung der Membrandichtung aus der Ruhestellung in die Funktionsstellung ausgebildet. Somit ist in der Funktionsstellung ein minimaler Fluidwiderstand für das Fluid gewährleistet, das im Fluidkanal strömt, da das Fluid nicht für die elastische Deformation der Membrandichtung sorgen muss. Diese Aufgabe wird vielmehr von der Kupplungseinrichtung wahrgenommen, die die Membrandichtung zumindest im Bereich der Ausweichvertiefung deformiert und hierdurch eine Änderung der Geometrie des in der Ruhestellung geschlossenen Fluidspalts herbeiführt. Vorzugsweise sind die Membrandichtung und die Kupplungsausnehmung zudem derart aufeinander abgestimmt, dass bei Entfernen der Kupplungseinrichtung aufgrund der Elastizitätseigenschaften der Membrandichtung eine Rückverformung der Membrandichtung stattfindet. Hierdurch werden ohne Einwirken weiterer äußerer Kräfte eine Rückkehr in die Ruhestellung und somit ein Verschluss der Kanalöffnung sowie eine Bereitstellung einer glatten und gut zu reinigenden Oberfläche gewährleistet. Dabei kann die Kupplungsausnehmung, die in der Oberfläche des Kupplungsgehäuses eingebracht ist, derart ausgebildet sein, dass die Membrandichtung oberflächenbündig mit der Oberfläche des Kupplungsgehäuses abschließt. Bevorzugt ist eine versenkte Anordnung der Membrandichtung vorgesehen, da hierdurch ein verbesserter Schutz gegenüber mechanischen Einflüssen und daraus resultierenden Beschädigungen der Membrandichtung gewährleistet werden kann.

Eine bevorzugte Ausführungsform der Kupplungskomponente sieht vor, dass sich der Fluidspalt prismatisch zwischen einer der Kanalöffnung abgewandten Oberseite der Membrandichtung und einer der Kanalöffnung zugewandten Unterseite der Membrandichtung erstreckt. Bei einer prismatischen Ausgestaltung des Fluidspalts weist dieser in zueinander parallelen Querschnittsebenen, die quer zu einem Abstand zwischen der Oberseite und der Unterseite ausgerichtet sind, stets einen konstanten Querschnitt auf. Vorzugsweise wird der Fluidspalt mit einem Schneidverfahren ohne Werkstoffabtrag in die, vorzugsweise einstückig ausgebildete, Membrandichtung eingebracht. Ein derartiges Schneidverfahren kann beispielsweise mit Hilfe eines Messers oder eines Schneidstempels durchgeführt werden, wobei im Zuge der Herstellung des Fluidspalts durch eine geeignete Geometrie und Bewegung des jeweiligen Schneidwerkzeugs nur ein minimaler, vorzugsweise kein, Werkstoffabtrag erfolgt. Durch diese Maßnahme weist die Membrandichtung in der Ruhestellung vorteilhafte Dichteigenschaften auf. Diese Dichteigenschaften können gegebenenfalls dadurch unterstützt werden, dass die Membrandichtung in Raumrichtungen quer zum Abstand zwischen der Oberseite und der Unterseite radial nach innen komprimiert wird, so dass einander gegenüberliegende Wandabschnitte des Fluidspalts mit einander entgegengesetzten Druckkräften beaufschlagt sind.

Bei einer bevorzugten Ausführungsform weist der Fluidspalt eine randseitige Entlastungsgeometrie, insbesondere eine H-förmige Profilierung, auf. Um eine randseitige Rissbildung am Fluidspalt zu vermeiden, ist an den einander entgegengesetzten Randbereichen des Fluidspalts eine Entlastungsgeometrie vorzusehen, mit deren Hilfe innere Spannungen in der Membrandichtung in günstiger Weise verteilt und damit reduziert werden können. Beispielhaft kann vorgesehen werden, den Fluidspalt randseitig mit Bohrungen zu versehen, deren Durchmesser sehr klein ist, um die Dichtwirkung der Membrandichtung nicht in Frage zu stellen. Besonders bevorzugt ist vorgesehen, dass der Fluidspalt eine H-förmige Profilierung aufweist. Mit einer derartigen Gestaltung des Fluidspalts gewährleisten die beiden seitlichen Schenkel der H-förmigen Profilierung des Fluidspalts eine Relativbeweglichkeit von vorgegebenen Bereichen der Membrandichtung. Bei einer Relativbewegung dieser vorgegebenen Bereiche der Membrandichtung zwischen der Ruhestellung und der Funktionsstellung wird ein Spaltquerschnitt für einen Bereich des Fluidspalts ausgehend von Null bis zu einem Maximalquerschnitt erweitert, wodurch ein Fluidspaltbereich, der die beiden Schenkel verbindet, in der Funktionsstellung aufgespreizt wird und damit den Fluidstrom durch die Membrandichtung ermöglicht.

Vorteilhaft ist es, wenn die Ausweichvertiefung spiegelsymmetrisch oder rotationssymmetrisch zu einer Mittelachse der Kanalöffnung ausgebildet ist. Dies ermöglicht in Verbindung mit einer möglichst zentrischen Positionierung des Fluidspalts über der Kanalöffnung und einer geeigneten Kupplungseinrichtung zur Überführung der Membrandichtung aus der Ruhestellung in die Funktionsstellung eine zumindest im wesentlichen symmetrische Öffnung des Fluidspalts und damit einen reibungsarmen Fluidstrom durch den Fluidspalt. Bevorzugt ist vorgesehen, dass sich die Ausweichvertiefung zumindest unterhalb von Endbereichen der Schenkel der H-förmigen Profilierung erstreckt.

Eine besonders bevorzugte Ausführungsform sieht vor, dass die Membrandichtung einen radial innenliegenden Membranbereich und einen daran angrenzenden, radial außenliegenden und randseitig umlaufenden, insbesondere ringförmigen, Verstärkungsabschnitt aufweist, der zur Festlegung der Membrandichtung in der Kupplungsausnehmung ausgebildet ist. Vorzugsweise ist der innenliegende Membranbereich folienartig oder plattenförmig mit einer vorzugsweise konstanten Materialstärke, insbesondere als planparalleler Bereich, ausgebildet. Dabei wird die Materialstärke des Membranbereichs in Abhängigkeit von den im Fluidkanal auftretenden Druckverhältnissen und/oder von den Materialeigenschaften der insbesondere aus einem gummielastischen Material hergestellten Membrandichtung und/oder von den bei einer Reinigung der Kupplungsausnehmung auftretenden Reinigungskräften gewählt. Exemplarisch ist vorgesehen, dass die Materialstärke des Membranbereichs in einem Intervall von 5 Prozent bis 25 Prozent des Abstands zwischen den beiden Schenkeln der H-förmigen Profilierung liegt. Hiermit kann ein vorteilhafter Kompromiss zwischen den Deformationseigenschaften der Membrandichtung und einem durch die Membrandichtung hervorgerufenen Fluidwiderstand bei Durchströmung des Fluidkanals mit einem Fluid erzielt werden. An den innenliegenden Membranbereich schließt sich radial außenliegend ein umlaufender Verstärkungsbereich an, der zum einen zur Stabilisierung des Membranbereichs dient und zum anderen für eine Festlegung der Membrandichtung in der Kupplungsausnehmung genutzt werden kann. Vorzugsweise ist der Verstärkungsbereich prismatisch ausgebildet und erstreckt sich in gleicher Richtung wie der Abstand zwischen der Oberseite und der Unterseite des Membranbereichs, wobei der Verstärkungsbereich in dieser Richtung über die Oberseite und/oder Unterseite des Membranbereichs hinausragen kann.

Bei einer vorteilhaften Weiterbildung der Erfindung ist vorgesehen, dass in der Kupplungsausnehmung benachbart zur Ausweichvertiefung ein umlaufender Montagekanal zur zumindest bereichsweisen Aufnahme des Verstärkungsabschnitts ausgebildet ist und dass der Verstärkungsabschnitt der Membrandichtung für eine abdichtende Aufnahme in dem Montagekanal ausgebildet ist. Mit Hilfe des Montagekanals kann eine kompakte Anordnung der Membrandichtung in der Kupplungsausnehmung gewährleistet werden.

Zweckmäßig ist, wenn die Ausweichvertiefung und der Montagekanal jeweils als ringförmige Bereiche der Kupplungsausnehmung koaxial zur Kanalöffnung ausgebildet sind. Hierdurch wird eine einfache Montage der Membrandichtung in der Kupplungsausnehmung gewährleistet, da nicht auf eine rotatorische Ausrichtung der Membrandichtung gegenüber der Kupplungsausnehmung geachtet werden muss.

Mit dem Kupplungsvorsprung wird bei der Anbringung der Kupplungseinrichtung an der Kupplungskomponente die für einen möglichst ungestörten Fluidstrom zwischen den jeweiligen Fluidkanälen erforderliche Überführung der Membrandichtung aus der Ruhestellung in die Funktionsstellung bewirkt. Dies geschieht durch die mechanische Einwirkung des Kupplungsvorsprungs, der bei der Anbringung der Kupplungseinrichtung an der Kupplungskomponente in die Kupplungsausnehmung eingreift und dabei die Membrandichtung bereichsweise deformiert. Dies führt zu einer Schwenkbewegung von Bereichen der Membrandichtung, wobei diese Bereiche in die Ausweichvertiefung ausweichen können. Vorzugsweise liegen die von der Kupplungseinrichtung auf die Membrandichtung übertragenen Kräfte innerhalb eines vorgebbaren Intervalls, das bei funktionsgerechter Abdichtwirkung für die Fluidkupplung insbesondere eine komfortable manuelle Bedienung durch einen Benutzer gewährleistet. Dabei kann eine Verbindungsbewegung zwischen der Kupplungseinrichtung und der Kupplungskomponente als lineare Steckbewegung oder als Schraubbewegung in Form einer Überlagerung einer linearen Steckbewegung mit einer Rotationsbewegung, die insbesondere um die Mittelachse der Kanalöffnung stattfindet, vorgenommen werden. Die Ausgestaltung der Kupplungseinrichtung mit dem Kupplungsvorsprung ist vorzugsweise derart gewählt, dass während einer Anbringung der Kupplungseinrichtung an der Kupplungskomponente eine fortdauernde fluidisch kommunizierende Verbindung zwischen den beiden Fluidkanälen unabhängig von einem in den Fluidkanälen herrschenden Fluiddruck gewährleistet ist. Bei Abnahme der Kupplungseinrichtung von der Kupplungskomponente findet aufgrund der Elastizitätseigenschaften der Membrandichtung eine selbsttätige Abdichtung der Kanalöffnung statt, so dass hierzu keine weiteren Maßnahmen oder technischen Einrichtungen erforderlich sind.

Bei der erfindungsgemäßen Fluidkupplung ist vorgesehen, dass der Kupplungsvorsprung umlaufend ausgebildet ist und für eine abdichtende Anlage an der Membrandichtung in einer Funktionsstellung ausgebildet ist, um eine kommunizierende Verbindung zwischen den Fluidkanälen in der Kupplungskomponente und in der Kupplungseinrichtung zu gewährleisten. Bei dieser Weiterbildung der Fluidkupplung ist vorteilhaft, dass der Membrandichtung hiermit eine Dreifachfunktion zukommt, so dass sie zusätzlich zu Abdichtung der Kanalöffnung in der Ruhestellung und zur Gewährleistung einer glatten, gut zu reinigenden Oberfläche in der Ruhestellung auch noch die Abdichtung zwischen den beiden Fluidkanälen der Kupplungseinrichtung und der Kupplungskomponente übernimmt. Diese Abdichtfunktion ergibt sich mit dem mechanischen Einwirken des Kupplungsvorsprungs auf die Membrandichtung, durch den die Membrandichtung aus der Ruhestellung in die Vorzugsstellung überführt wird. Hierbei tritt eine vorgebbare axiale Kompression der Membrandichtung auf, die von der Geometrie des Membranbereichs sowie der Ausweichausnehmung und des Kupplungsvorsprungs abhängt und durch die vorhandene Spalte zwischen Kupplungsvorsprung und Kupplungskomponente abgedichtet werden.

Eine vorteilhafte Ausführungsform der Erfindung ist in der Zeichnung dargestellt. Hierbei zeigt:
- Figur 1: eine schematische, perspektivische Darstellung einer Fluidkupplung mit einer Kupplungskomponente, einer Kupplungseinrichtung und einer zu Montage in die Kupplungskomponente vorgesehenen Membrandichtung,
- Figur 2: eine Draufsicht auf die Membrandichtung gemäß der Figur 1,
- Figur 3: eine Ansicht von unten auf die Membrandichtung gemäß der Figur 1,
- Figur 4: eine Schnittdarstellung der Fluidkupplung gemäß der Figur 1, wobei die Membrandichtung in der Kupplungskomponente montiert ist und sich in der Ruhestellung befindet, da die Kupplungseinrichtung beabstandet von der Kupplungskomponente angeordnet ist, und
- Figur 5: eine Schnittdarstellung der Fluidkupplung gemäß der Figur 1, wobei die Membrandichtung in der Kupplungskomponente montiert ist und sich in der Funktionsstellung befindet, da die Kupplungseinrichtung an der Kupplungskomponente angebracht ist.

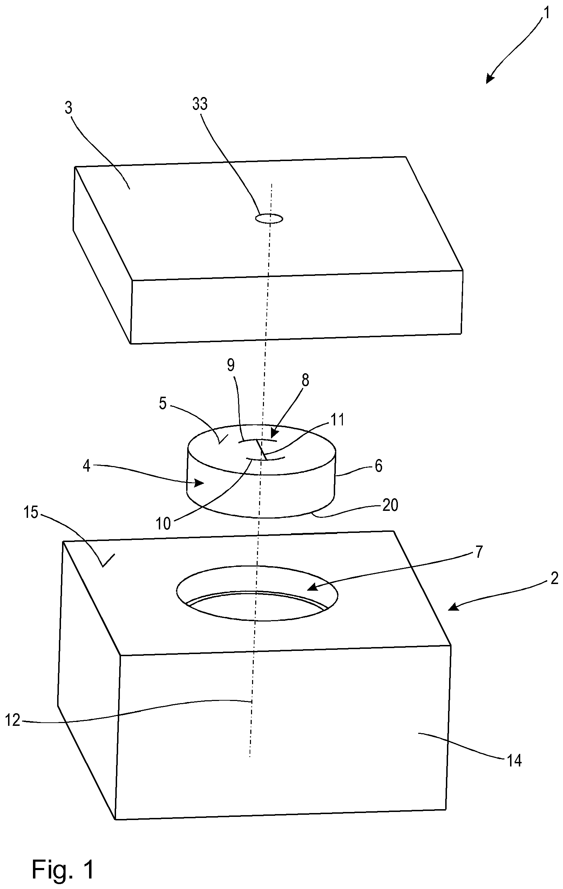

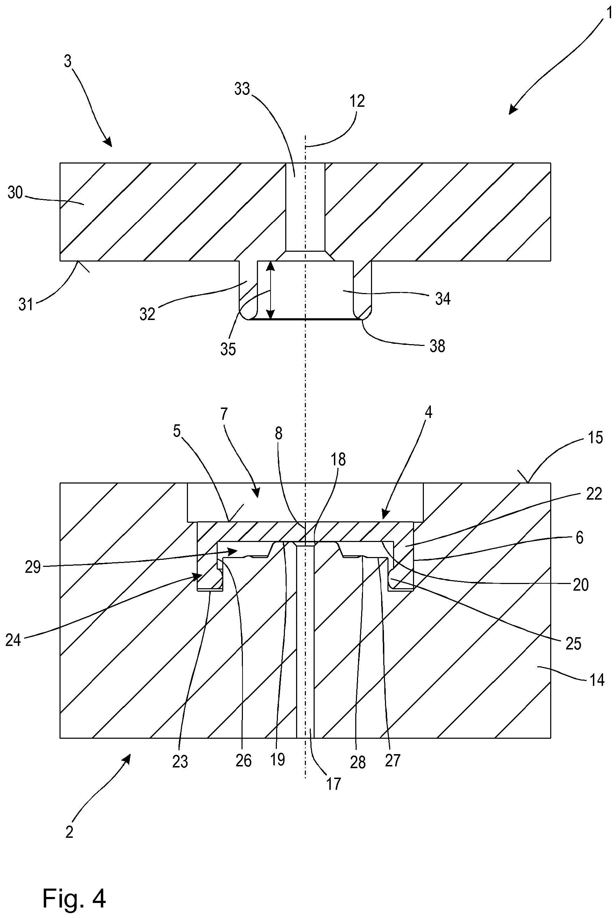

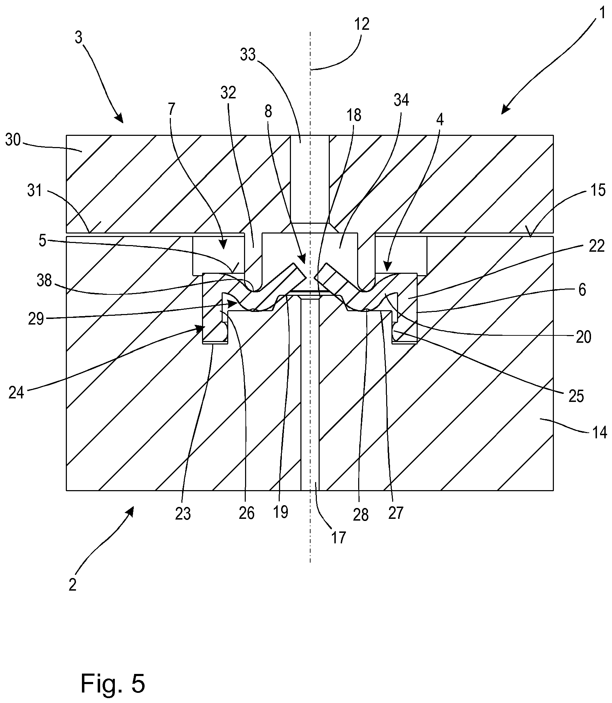

Eine rein schematisch in der Figur 1 dargestellte Fluidkupplung 1 umfasst eine Kupplungskomponente 2 und eine Kupplungseinrichtung 3. Die Kupplungskomponente 2 und die Kupplungseinrichtung 3 sind beispielsweise nicht näher dargestellten Vorrichtungen zugehörig, die mit Hilfe der Fluidkupplung ein fluidisch miteinander gekoppelt werden sollen. Beispielsweise kann die Kupplungskomponente 2 als Bestandteil einer Fluidfördereinrichtung, insbesondere einer Fluidpumpe, ausgebildet sein, die zur Bereitstellung eines Fluids aus einem nicht dargestellten Vorratsbehälter ausgebildet ist. In diesem Fall kann die Kupplungseinrichtung 3 beispielhaft als Fluidverbraucher ausgebildet sein, dem von der Kupplungskomponente 2 Fluid zugeführt werden kann. In der Darstellung der Figur 1 ist eine der Kupplungskomponente 2 zugehörige Membrandichtung 4 in einer Demontagestellung dargestellt. Somit ist ersichtlich, dass die Membrandichtung 4 exemplarisch eine kreisförmige Oberfläche 5 sowie eine kreisringförmigen Umfangsfläche 6 aufweist. Wie aus den nachstehend näher beschriebenen Figuren 4 und 5 entnommen werden kann, wird die Membrandichtung 4 in einer Kupplungsausnehmung 7 der Kupplungskomponente 2 montiert, um die gewünschten, nachstehend näher beschriebenen Funktionen wahrnehmen zu können.

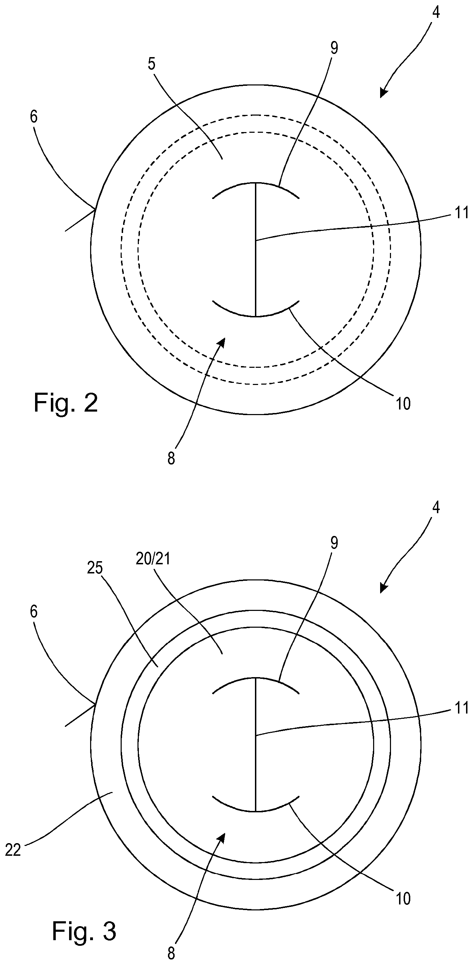

In der Draufsicht gemäß der Figur 2 sowie in der Ansicht von unten gemäß der Figur 3 ist der H-förmig profilierte, prismatische Fluidspalt 8 zu erkennen, der beispielhaft mit einem nicht dargestellten Schneidstempel in die Membrandichtung 4 geschnitten wird, ohne dass dabei ein wesentlicher Materialabtrag an der Membrandichtung 4 stattfindet. Dadurch wird gewährleistet, dass der Fluidspalt 8 in der Ruhestellung, wie sie in den Figuren 2 und 3 dargestellt ist, keinen freien Querschnitt aufweist, der einen Durchtritt von Fluid längs des Fluidspalts 8 ermöglichen würde. Ein derartiger Fluiddurchtritt mit wahlweise dadurch ermöglicht, dass die Membrandichtung 4 lokal deformiert wird oder dass über der Membrandichtung 4 ein fluidischer Druckgradient anliegt, der eine elastische Deformation des Materials der Membrandichtung 4 bewirkt und damit ein Öffnen des Fluidspalts 8 hervorruft. Exemplarisch umfasst der H-förmig profilierte, prismatische Fluidspalt 8 zwei jeweils bogenförmig gekrümmt ausgebildete Schenkel 9, 10, die durch eine Verbindungslinie 11 miteinander verbunden sind, wobei die Verbindungslinie 11 exemplarisch eine Mittelsenkrechte für die beiden Schenkel 9 und 10 bildet. Vorzugsweise weist der Fluidspalt 8 längs einer senkrecht zur Darstellungsebene der Figur 2 ausgerichteten, in den Figuren 3 und 4 dargestellten Abstandsachse 12 eine konstante Profilierung auf.

Wie der Darstellung der Figuren 4 und 5 entnommen werden kann, ist in einem Kupplungsgehäuse 14 der Kupplungskomponente 2 ausgehend von einer Oberfläche 15 eine Kupplungsausnehmung 7 eingebracht, die rein exemplarisch rotationssymmetrisch zur Abstandsachse 12 ausgebildet ist. Ferner ist beispielhaft vorgesehen, dass die Abstandsachse 12 als Mittelachse eines Fluidkanals 17 dient, der in der Kupplungskomponente 2 ausgebildet ist und der mit einer Kanalöffnung 18 an einer Stirnfläche 19 der Kupplungsausnehmung 7 ausmündet. Die Stirnfläche 19 ist kreisringförmig ausgebildet und exemplarisch parallel zur Oberfläche 15 ausgerichtet. Die Membrandichtung 4 ist derart in der Kupplungsausnehmung 7 angeordnet, dass sie in der Ruhestellung, wie in der Figur 4 dargestellt, mit einer Unterseite 20 flächig an der Stirnfläche 19 anliegt und den Fluidkanal 17 flächig abdichtet. Die Membrandichtung 4 kann in einen rein exemplarisch kreisförmig ausgebildeten Membranbereich 21 und in einen radial außenliegend an den Membranbereich 21 einstückig angeformten Verstärkungsabschnitt 22 unterteilt werden. Dabei weist der Membranbereich 21 eine konstante Materialstärke auf, die in Abhängigkeit von den Elastizitätseigenschaften und den zu erwartenden Druckverhältnissen an der Membrandichtung 4 gewählt ist, um beispielsweise ohne Einwirkung der Kupplungseinrichtung 3 stets eine zuverlässige Abdichtung des Fluidkanals 17 zu gewährleisten. Der ringförmig umlaufend ausgebildete Verstärkungsabschnitt 22 dient zur Festlegung der Membrandichtung 4 in der Kupplungsausnehmung 7. Hierzu ist in der Kupplungsausnehmung 7 rein exemplarisch koaxial zur Abstandsachse 12 ein ringförmig umlaufender Montagekanal 23 ausgebildet, in den ein Endbereich 24 des Verstärkungsabschnitts 22 montiert werden kann. Um eine Abdichtung zwischen der Membrandichtung 4 und dem Montagekanal 23 zu gewährleisten, ist der Endbereich 24 rein exemplarisch mit einem radial nach innen weisenden, umlaufenden und einstückig angeformten Dichtwulst 25 versehen.

Die Membrandichtung 4 und die Kupplungsausnehmung 7 begrenzen ein Raumvolumen, dass zumindest bereichsweise für eine Ausweichbewegung der Membrandichtung 4 bei einer Überführung in den Funktionszustand, wie er in der Figur 5 dargestellt ist, dient. Im Wesentlichen wird dieses Raumvolumen von der kreisförmigen Unterseite 20 der Membrandichtung 4, einer kreisringförmigen Innenoberfläche 26 des Verstärkungsabschnitts 22 und einer gegenüber der Stirnfläche 19 axial zurückversetzen Ringfläche 27 begrenzt. Rein exemplarisch ist die Ringfläche 27 mit einem ringförmigen Vorsprung 28 versehen, der von der Ringfläche 27 in Richtung der Oberfläche 15 abragt und der zur Unterstützung einer Abdichtungswirkung zwischen der Membrandichtung 4 und der Kupplungseinrichtung 3 ausgebildet ist.

Dieses Raumvolumen wird auch als Ausweichvertiefung 29 bezeichnet, da es eine Ausweichbewegung von Bereichen der Membrandichtung 4 bei einer Montage der Kupplungseinrichtung 3 an die Kupplungskomponente 2 ermöglicht.

Die in den Figuren 4 und 5 nur rein schematisch dargestellte Kupplungseinrichtung 3 umfasst einen Grundkörper 30, der eine geometrisch zur Oberfläche 15 korrespondierende Kupplungsfläche 31 aufweist, die für eine flächige Anlage an der Oberfläche 15 ausgebildet ist. Von der Kupplungsfläche 31 ragt ein ringförmiger Kupplungsvorsprung 32 ab, dessen Durchmesser rein exemplarisch dem Durchmesser des Vorsprungs 28 in der Kupplungsausnehmung 7 entspricht. Ferner wird der Grundkörper 30 von einem Fluidkanal 33 durchsetzt, der in einem Innenbereich 34 des Kupplungsvorsprungs 32 ausmündet. Eine axiale Erstreckung 35 des Kupplungsvorsprungs 32 längs der Abstandsachse 12 ist derart gewählt, dass der Kupplungsvorsprung 32 bei flächiger Anlage der Kupplungsfläche 31 an der Oberfläche 15 die Membrandichtung 4 bereichsweise deformieren und bereichsweise in die Ausweichvertiefung 29 verdrängen kann. Im Zuge dieser Verdrängungsbewegung, die auch als Überführung der Membrandichtung 4 aus dem Ruhezustand in den Funktionszustand angesehen werden kann, werden Membranbereiche 36, 37, die zwischen Schenkeln 9, 10 des Fluidspalts 8 angeordnet sind, bogenförmig deformiert, wie dies in der Figur 5 dargestellt ist. Hierdurch wird der Fluidspalt 8 im Bereich der Verbindungslinie 11 aufgespreizt und gibt einen Fluidquerschnitt frei, durch den eine fluidisch kommunizierende Verbindung zwischen dem Fluidkanal 17 und dem Fluidkanal 33 ermöglicht wird.

Bei geeigneter Auslegung der axialen Erstreckung 35 des Kupplungsvorsprungs 32 wird bei der Montage der Kupplungseinrichtung 3 an die Kupplungskomponente 2 zusätzlich zur bogenförmigen Deformation der Membranbereiche 36, 37 eine vollständig umlaufende Anlage einer ringförmigen Stirnfläche 38 an der Membrandichtung 4 sowie der Membrandichtung 4 am Vorsprung 28 bewirkt, so dass eine vollständige fluidische Abdichtung für ein aus den beiden Fluidkanälen 17 und 33 gebildetes Fluidkanalsystem gewährleistet ist. Dies stellt neben der Abdichtung der Kanalöffnung 18 im Ruhezustand und der Bereitstellung einer gut zu reinigenden Oberfläche der Kupplungsausnehmung 7 im Ruhezustand der Membrandichtung 4 eine zusätzliche Funktion dar, die die Membrandichtung 4 beim Zusammenspiel der Kupplungskomponente 2 mit der Kupplungseinrichtung 3 hat.

## Patentansprüche

1. Fluidkupplung zur lösbaren Verbindung von fluidführenden Komponenten, insbesondere fluidführenden Komponenten in medizinischen Geräten, mit einer Kupplungskomponente (2) sowie mit einer Kupplungseinrichtung (3),
wobei die Kupplungskomponente (2) ein Kupplungsgehäuse (14) aufweist, das bereichsweise von einem Fluidkanal (17) durchsetzt ist und das mit einer Kupplungsausnehmung (7) versehen ist, die vertieft in eine Oberfläche (15) des Kupplungsgehäuses (14) eingebracht ist, wobei an einer Stirnfläche (19) der Kupplungsausnehmung (7) eine Kanalöffnung (18) des Fluidkanals (17) ausmündet, und die Kanalöffnung (18) von einer Membrandichtung (4) aus einem elastischen Material überdeckt ist, die von einem Fluidspalt (8) durchsetzt ist, der derart ausgebildet ist, dass die Membrandichtung (4) in einer Ruhestellung die Kanalöffnung (18) abdichtet und einer Funktionsstellung die Kanalöffnung (18) freigibt, wobei in der Kupplungsausnehmung (7) benachbart zur Kanalöffnung (18) wenigstens eine Ausweichvertiefung (29) ausgebildet ist, die für eine Ausweichbewegung eines Bereichs (36, 37) der Membrandichtung (4) bei einem Übergang der Membrandichtung (4) aus der Ruhestellung in die Funktionsstellung ausgebildet ist,
wobei die Kupplungseinrichtung (3) einen Kupplungsvorsprung (32) aufweist, der für einen Eingriff in die Kupplungsausnehmung (7) ausgebildet ist, wobei der Kupplungsvorsprung (32) von einem Fluidkanal (33) durchsetzt ist und für eine Auslenkung eines Bereichs (36, 37) der Membrandichtung (4) in die Ausweichvertiefung (29) ausgebildet ist, und der Kupplungsvorsprung (32) umlaufend ausgebildet ist und für eine abdichtende Anlage an der Membrandichtung (4) in einer Funktionsstellung ausgebildet ist, um eine kommunizierende Verbindung zwischen den Fluidkanälen (17, 33) in der Kupplungskomponente (2) und in der Kupplungseinrichtung (3) zu gewährleisten,
**dadurch gekennzeichnet, dass**
die Kupplungseinrichtung (3) zur Anbringung an der Oberfläche (15) des Kupplungsgehäuses (14) ausgebildet ist, und
der Kupplungsvorsprung (32), die Membrandichtung (4) und die Ausweichvertiefung (29) derart ausgebildet sind, dass in einer Funktionsstellung der Kupplungsvorsprung (32) gegenüber der Membrandichtung (4) abgedichtet ist und die Membrandichtung (4) gegenüber einer gegenüber der Stirnfläche (19) der Kupplungsausnehmung (7) axial zurückversetzten Anlagefläche (27) in der Ausweichvertiefung (29) abgedichtet ist, wobei die Anlagefläche in der Ausweichvertiefung (29) eine Ringfläche (27) ist.

2. Fluidkupplung nach Anspruch 1, **dadurch gekennzeichnet, dass** sich der Fluidspalt (8) prismatisch zwischen einer der Kanalöffnung (18) abgewandten Oberseite der Membrandichtung (4) und einer der Kanalöffnung (18) zugewandten Unterseite (20) der Membrandichtung (4) erstreckt, wobei der Fluidspalt (8) in zueinander parallelen Querschnittsebenen, die quer zu einem Abstand zwischen der Oberseite und der Unterseite der Membrandichtung (4) ausgerichtet sind, stets einen konstanten Querschnitt aufweist.

3. Fluidkupplung nach Anspruch 1, **dadurch gekennzeichnet, dass** der Fluidspalt (8) eine randseitige Entlastungsgeometrie, insbesondere eine H-förmige Profilierung, aufweist.

4. Fluidkupplung nach Anspruch 1, 2 oder 3, **dadurch gekennzeichnet, dass** die Ausweichvertiefung (29) spiegelsymmetrisch oder rotationssymmetrisch zu einer Mittelachse (12) der Kanalöffnung (18) ausgebildet ist.

5. Fluidkupplung nach Anspruch 1, 2, 3 oder 4, **dadurch gekennzeichnet, dass** die Membrandichtung (4) einen radial innenliegenden Membranbereich (21) und einen daran angrenzenden, radial außenliegenden und randseitig umlaufenden, insbesondere ringförmigen, Verstärkungsabschnitt (22) aufweist, der zur Festlegung der Membrandichtung (4) in der Kupplungsausnehmung (7) ausgebildet ist.

6. Fluidkupplung nach Anspruch 5, **dadurch gekennzeichnet, dass** in der Kupplungsausnehmung (7) benachbart zur Ausweichvertiefung (29) ein umlaufender Montagekanal (23) zur zumindest bereichsweisen Aufnahme des Verstärkungsabschnitts (22) ausgebildet ist und dass der Verstärkungsabschnitt (22) der Membrandichtung (4) für eine abdichtende Aufnahme in dem Montagekanal (23) ausgebildet ist.

7. Fluidkupplung nach Anspruch 6, **dadurch gekennzeichnet, dass** die Ausweichvertiefung (29) und der Montagekanal (23) jeweils als ringförmige Bereiche der Kupplungsausnehmung (7) koaxial zur Kanalöffnung (18) ausgebildet sind.

8. Fluidkupplung nach einem der Ansprüche 1 bis 7, **dadurch gekennzeichnet, dass** der Kupplungsvorsprung (32) eine ringförmige Stirnfläche (38) aufweist.

## Claims

1. Fluid coupling for releasably connecting fluid-conveying components, in particular fluid-conveying components in medical equipment, having a coupling component (2) and a coupling device (3),
the coupling component (2) comprising a coupling housing (14) through which a fluid channel (17) passes in regions and which is provided with a coupling recess (7) that is sunken into a surface (15) of the coupling housing (14), a channel opening (18) of the fluid channel (17) ending at an end face (19) of the coupling recess (7), and the channel opening (18) is covered by a membrane seal (4) which is made of a resilient material and through which a fluid gap (8) passes that is designed such that the membrane seal (4), when in a resting position, seals the channel opening (18) and, when in an operative position, opens the channel opening (18), at least one yielding depression (29) being formed in the coupling recess (7) adjacently to the channel opening (18), which depression is designed for a yielding movement of a region (36, 37) of the membrane seal (4) when the membrane seal (4) transitions from the resting position into the operative position,
the coupling device (3) comprising a coupling projection (32) that is designed to engage in the coupling recess (7), wherein a fluid channel (33) passes through the coupling projection (32), which coupling projection is designed to deflect a region (36, 37) of the membrane seal (4) into the yielding depression (29), and the coupling projection (32) being designed to extend circularly and to sealingly abut the membrane seal (4) when in an operative position in order to ensure a communicating connection between the fluid channels (17, 33) in the coupling component (2) and in the coupling device (3),
**characterized in that**
the coupling device (3) is designed to be attached to the surface (15) of the coupling housing (14), and
the coupling projection (32), the membrane seal (4) and the yielding depression (29) are designed such that the coupling projection (32) is sealed with respect to the membrane seal (4) when in an operative position, and the membrane seal (4) is sealed with respect to an abutment surface (27) in the yielding depression (29), the abutment surface (27) being axially set back with respect to the end face (19) of the yielding depression (29), the abutment surface in the yielding depression (29) being an annular face (27).

2. Fluid coupling according to claim 1, **characterised in that** the fluid gap (8) extends prismatically between an upper side of the membrane seal (4) facing away from the channel opening (18) and a lower side (20) of the membrane seal (4) facing the channel opening (18), the fluid gap (8) always has a constant cross section in cross-sectional planes that are parallel to one another and oriented transversely to a space between the upper side and the lower side.

3. Fluid coupling according to claim 1, **characterised in that** the fluid gap (8) has a strain-relieving geometry on the edges thereof, in particular an H-shaped profiling.

4. Fluid coupling according to claims 1, 2 or 3, **characterised in that** the yielding depression (29) is formed in mirror symmetry or rotational symmetry with respect to a central axis (12) of the channel opening (18).

5. Fluid coupling according to claims 1, 2, 3 or 4, **characterised in that** the membrane seal (4) has a radially internal membrane region (21) and a reinforcing portion (22) that adjoins said region, is radially external, extends round the edge of said seal, in particular is annular, and is designed to fix the membrane seal (4) in the coupling recess (7).

6. Fluid coupling according to claim 5, **characterised in that** a circumferential mounting channel (23) for receiving the reinforcing portion (22) at least in regions is formed in the coupling recess (7) adjacently to the yielding depression (29), and **in that** the reinforcing portion (22) of the membrane seal (4) is designed to be sealingly received in the mounting channel (23).

7. Fluid coupling according to claim 6, **characterised in that** the yielding depression (29) and the mounting channel (23) are each designed as annular regions of the coupling recess (7) that are coaxial with the channel opening (18).

8. Fluid coupling according to one of claims 1 to 7, **characterised in that** the coupling projection (32) has an annular end face (38).

## Revendications

1. Raccord pour fluide pour la liaison amovible de composants acheminant du fluide, en particulier de composants acheminant du fluide dans des appareils médicaux, avec un composant de raccord (2) ainsi qu'avec un dispositif de raccord (3),
dans lequel le composant de raccord (2) présente un boîtier de raccord (14) qui est traversé par endroits par un canal de fluide (17) et qui est pourvu d'un évidement de raccord (7) qui est introduit en creux dans une surface (15) du boîtier de raccord (14), dans lequel une ouverture de canal (18) du canal de fluide (17) débouche sur une surface avant (19) de l'évidement de raccord (7), et l'ouverture de canal (18) est recouverte par une garniture de membrane (4) en un matériau élastique qui est traversée par une fente de fluide (8) qui est réalisée de telle manière que la garniture de membrane (4) rende étanche dans une position de repos l'ouverture de canal (18) et libère dans une position fonctionnelle l'ouverture de canal (18), dans lequel au moins une cavité de déport (29) est réalisée dans l'évidement de raccord (7) de manière contiguë à l'ouverture de canal (18), laquelle cavité de déport (29) est réalisée pour un mouvement de déport d'une zone (36, 37) de la garniture de membrane (4) lors d'une transition de la garniture de membrane (4) de la position de repos dans la position fonctionnelle,
dans lequel le dispositif de raccord (3) présente une saillie de raccord (32) qui est réalisée pour un engagement dans l'évidement de raccord (7), dans lequel la saillie de raccord (32) est traversée par un canal de fluide (33) et est réalisée pour une déviation d'une zone (36, 37) de la garniture de membrane (4) dans la cavité de déport (29), et la saillie de raccord (32) est réalisée de manière tournante et est réalisée pour un appui étanche contre la garniture de membrane (4) dans une position fonctionnelle afin de garantir une liaison de communication entre les canaux de fluide (17, 33) dans le composant de raccord (2) et dans le dispositif de raccord (3),
**caractérisé en ce que**
le dispositif de raccord (3) est réalisé pour le montage sur la surface (15) du boîtier de raccord (14), et
la saillie de raccord (32), la garniture de membrane (4) et la cavité de déport (29) sont réalisées de telle manière que dans une position fonctionnelle, la saillie de raccord (32) soit rendue étanche par rapport à la garniture de membrane (4) et la garniture de membrane (4) soit rendue étanche par rapport à une surface d'appui (27) en déport axialement par rapport à la surface avant (19) de l'évidement de raccord (7) dans la cavité de déport (29), dans lequel la surface d'appui dans la cavité de déport (29) est une surface annulaire (27).

2. Raccord pour fluide selon la revendication 1, **caractérisé en ce que** la fente de fluide (8) s'étend en prisme entre un côté supérieur éloigné de l'ouverture de canal (18) de la garniture de membrane (4) et un côté inférieur (20) tourné vers l'ouverture de canal (18) de la garniture de membrane (4), dans lequel la fente de fluide (8) présente dans des plans de section parallèles l'un à l'autre qui sont orientés transversalement à une distance entre le côté supérieur et le côté inférieur de la garniture de membrane (4), toujours une section transversale constante.

3. Raccord pour fluide selon la revendication 1, **caractérisé en ce que** la fente de fluide (8) présente une géométrie de décharge côté bord, en particulier un profilage en forme de H.

4. Raccord pour fluide selon la revendication 1, 2 ou 3, **caractérisé en ce que** la cavité de déport (29) est réalisée de manière symétrique en miroir ou symétrique en rotation à un axe médian (12) de l'ouverture de canal (18).

5. Raccord pour fluide selon la revendication 1, 2, 3 ou 4, **caractérisé en ce que** la garniture de membrane (4) présente une zone de membrane (21) radialement intérieure et une section de renforcement (22) contiguë à celle-ci, radialement extérieure et tournant côté bord, en particulier annulaire qui est réalisée pour la fixation de la garniture de membrane (4) dans l'évidement de couplage (7).

6. Raccord pour fluide selon la revendication 5, **caractérisé en ce que** dans l'évidement de raccord (7) de manière contigüe à la cavité de déport (29), un canal de montage (23) périphérique est réalisé pour la réception au moins par endroits de la section de renforcement (22) et que la section de renforcement (22) de la garniture de membrane (4) est réalisée pour un logement étanche dans le canal de montage (23).

7. Raccord pour fluide selon la revendication 6, **caractérisé en ce que** la cavité de déport (29) et le canal de montage (23) sont réalisés respectivement comme zones annulaires de l'évidement de raccord (7) coaxialement à l'ouverture de canal (18).

8. Raccord pour fluide selon l'une des revendications 1 à 7, **caractérisé en ce que** la saillie de raccord (32) présente une surface avant (38) annulaire.
